# EUROPÄISCHE PATENTANMELDUNG

(11) **EP 0 552 759 A1**
(43) Veröffentlichungstag der Anmeldung: **28.07.1993**
(21) Anmeldenummer: 93100884.1
(22) Anmeldetag: 21.01.1993
(51) Int. Cl.: C07D 239/52

(54) **Verfahren zur Herstellung von 4,6-Dialkoxypyrimidinen**

(30) Priorität: 23.01.1992 CH 188/92
(71) Anmelder: LONZA AG, CH-3945 Gampel/Wallis (CH)
(72) Erfinder: Escher, André, Dr., Glis (Kanton Wallis) (CH); Previdoli, Felix, Dr., Brig (Kanton Wallis) (CH)
(74) Vertreter: Weinhold, Peter, Dr.

(57) **Zusammenfassung**

Beschrieben wird ein Verfahren zur Herstellung von 4,6-Dialkoxypyrimidinen der allgemeinen Formel 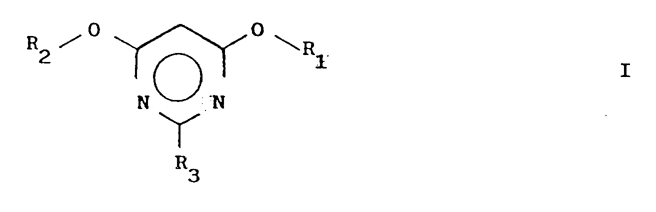
ausgehend von einem Propandiimidat der allgemeinen Formel 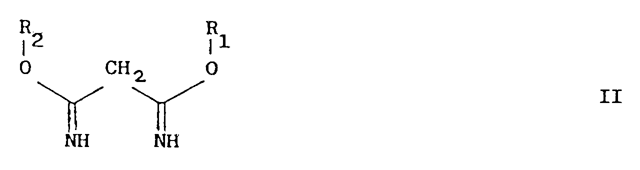
und einem Anhydrid der allgemeinen Formel

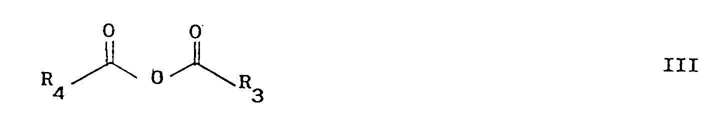

## Beschreibung

Die Erfindung betrifft ein neues Verfahren zur Herstellung von 4,6-Dialkoxypyrimidinen der allgemeinen Formel 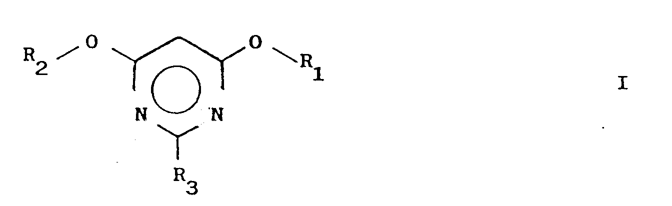
worin R₁ und R₂ eine C₁-C₄-Alkylgruppe und R₃ ein Wasserstoffatom oder eine C₁-C₄-Alkylgruppe bedeuten, ausgehend von einem Propandiimidat und einem Anhydrid.

Diese 4,6-Dialkoxypyrimidine können durch bekannte Reaktionen, z.B. durch Nitrierung mit anschliessender Reduktion, in die 5-Aminopyrimidine überführt werden, welche beispielsweise wichtige Zwischenprodukte zur Herstellung von Herbiziden darstellen (EP-PS 195 259).

Bekannt ist die Herstellung von in 2-Stellung unsubstituiertem 4,6-Dimethoxypyrimidin(Shepherd et al., J. Org. Chem., 1961, 26, S. 2764 - 2769) und die Herstellung von 2-Methyl-4,6-dimethoxypyrimidin (M. Prystas, Coll. Czech. Chem. Comm., 1967, 32, S. 4241).
Bei diesen beiden Verfahren werden die entsprechenden 4,6-Dialkoxypyrimidine, ausgehend von 4,6-Dichlorpyrimidinen durch Substitution mit Methylat hergestellt.

Ein grosser Nachteil dieser beiden Verfahren besteht darin, dass zunächst die 4,6-Dichlorpyrimidine ausgehend von den entsprechenden Dihydroxypyrimidinen mit chlorierten Phosphatverbindungen synthetisiert werden müssen, wobei grosse Mengen Phosphatabfälle anfallen.

Aufgabe der Erfindung war, diese Nachteile zu beseitigen und ein einfaches, ökologisches und wirtschaftlich gangbares Verfahren zur Herstellung von 2-substituierten und von 2-unsubstituierten 4,6-Dialkoxypyrimidinen zur Verfügung zu stellen.

Diese Aufgabe wurde mit dem neuen Verfahren gemäss Patentanspruch 1 gelöst.

Erfindungsgemäss wird das Verfahren derart durchgeführt, dass man ein Propandiimidat der Formel 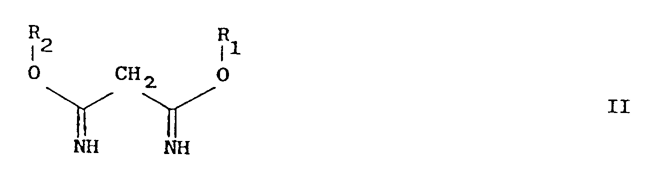
worin R₁ und R₂ die genannte Bedeutung haben mit einem Anhydrid der allgemeinen Formel 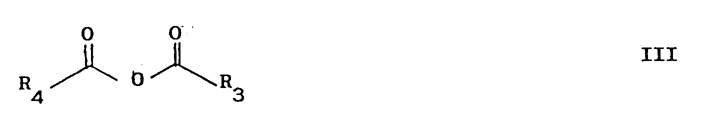
worin R₃ die genannte Bedeutung hat und R₄ eine C₁-C₄-Alkylgruppe bedeuten, in das Produkt gemäss Formel I überführt.

Propandiimidat, als Edukt für das Verfahren, kann ausgehend von einem Propandiimidat-Dihydrochlorid auf einfache Weise gemäss der EP-PS 024 200 hergestellt werden. Propandiimidat-Dihydrochlorid kann z.B. auf einfache Weise gemäss K. Hartke & H. G. Müller, Arch. Pharm. (Weinheim), 1988, 321, S. 863 - 871 synthetisiert werden.

Zweckmässig wird als Propandiimidat Dimethyl-1,3-propandiimidat, worin R₁ und R₂ eine Methylgruppe bedeuten, für das Verfahren eingesetzt.

Die Anhydride sind käuflich oder können aus der entsprechenden Säure nach Standardverfahren hergestellt werden.

Wird Formylacetat als Anhydrid für das Verfahren eingesetzt, kann dieses beispielsweise gemäss Muramatsu et al., Bull. Chem. Soc. Jpn., 1965, 38, S. 244 hergestellt werden.

Die zweckmassigen Anhydride für das Verfahren sind z.B.: Formylacetat, worin R₃ ein Wasserstoffatom und R₄ eine Methylgruppe bedeutet und Essigsäureanhydrid, worin R₃ und R₄ jeweils eine Methylgruppe bedeuten.

Zweckmässig wird das Propandiimidat und das Anhydrid in äquimolaren Mengen eingesetzt.

Zweckmässig wird die Umsetzung in Gegenwart einer Base, wie beispielsweise Ammoniak oder anderen Aminen durchgeführt, vorzugsweise in Gegenwart von Ammoniak.

Die Umsetzung wird zweckmassig bei einer Temperatur von 0 bis 100°C, vorzugsweise von 0 bis 40°C, durchgeführt.

Zweckmässig wird die Umsetzung bei einem pH-Wert von 5 bis 8, vorzugsweise von 6 bis 7, durchgeführt.

Als Lösungsmittel sind für das Verfahren inerte Lösungsmittel geeignet, wie beispielsweise Diethylether, Methylenchlorid, Acetonitril, Toluol oder Gemische von diesen. Vorzugsweise werden nicht wassermischbare Lösungsmittel eingesetzt, wie beispielsweise Methylenchlorid, Diethylether oder eine Mischung von diesen.

Nach einer üblichen Umsetzung von 1 bis 4 h kann dann das Produkt gemäss Formel I mittels fachmännisch üblichen Methoden aufgearbeitet werden.

### Beispiel 1:

### 4,6-Dimethoxypyrimidin

5,0 g Dimethyl-1,3-propandiimidat-Dihydrochlorid wurde unter kräftigem Rühren zu einem Gemisch von 25 ml CH₂Cl₂ und 25 ml einer wässrigen K₂CO₃-Lösung (300 g K₂CO₃/l Lösung) gegeben. Nach 5 min wurde die organische Phase abgetrennt und die Wasserphase mit 10 ml CH₂Cl₂ extrahiert. Die vereinigten organischen Phasen wurden über Na₂SO₄ getrocknet und filtriert. Ein frisch bereitetes Gemisch von 2,5 g Formylacetat (hergestellt aus Acetylchlorid und Natriumformiat gemäss Muramatsu et al., Bull. Chem. Soc. Jpn, 1965, 38, S. 244) in 2 ml Diethylether wurde bei 0°C zur obigen Lösung des Diimidats gegeben und während zwei Stunden bei dieser Temperatur gerührt. Man leitete eine kleine Menge Ammoniakgas ein (oder fügte etherische Ammoniaklösung hinzu), so dass das Reaktionsgemisch mit angefeuchtetem pH-Papier eine etwa neutrale Reaktion zeigte. Nach einer weiteren Stunde Rühren bei 0°C wurden 10 ml Wasser hinzugefügt. Die organische Phase wurde abgetrennt, über Na₂SO₄ getrocknet und schonend eingeengt. Nach Destillation im Kugelrohrofen (Produktfraktion: 110°C/16 mbar) erhielt man das Produkt als farbloses Öl, das beim Stehenlassen allmählich erstarrte. Ausbeute: 2,4 g = 66,3% bezogen auf das eingesetzte Dihydrochlorid bei einem Produktgehalt von 96% (GC).

### Beispiel 2:

### 4,6-Dimethoxy-2-methylpyrimidin

Wie bei Beispiel 1 wurden 5 g Dimethyl-1,3-propandiimidat-Dihydrochlorid behandelt und in CH₂Cl₂ aufgenommen. Nach Zugabe von 2,6 g Essigsäureanhydrid wurde die Lösung während zwei Stunden rückflussiert. Nach Zudosieren von NH₃ bis zur neutralen Reaktion mit angefeuchtetem pH-Papier rückflussierte man eine weitere Stunde, liess abkühlen und fügte 5 ml Wasser hinzu. Nach Abdestillieren von CH₂Cl₂ am Rotationsverdampfer wurde das ausgefallende Produkt abfiltriert, mit wenig Wasser gewaschen und bei Raumtemperatur bei 26 mbar getrocknet. Man erhielt 2,2 g weisses kristallines Produkt (Smp: 51 - 52°C) mit einem Gehalt von 98% (GC) was einer Ausbeute von 56,3% bezogen auf das Dihydrochlorid entsprach.
Durch Extraktion der Wasserphase und anschliessende Säulenchromatographie liess sich die Ausbeute auf 68% erhöhen.

## Patentansprüche

1. Verfahren zur Herstellung von 4,6-Dialkoxypyrimidinen der allgemeinen Formel 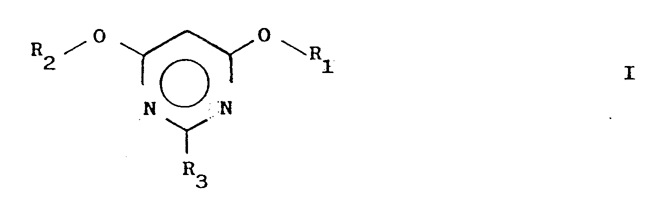 worin R₁ und R₂ eine C₁-C₄-Alkylgruppe und R₃ ein Wasserstoffatom oder eine C₁-C₄-Alkylgruppe bedeuten, dadurch gekennzeichnet, dass man ein Propandiimidat der allgemeinen Formel 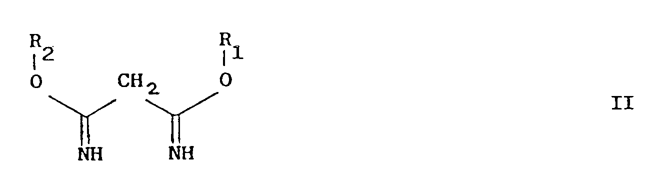 worin R₁ und R₂ die genannte Bedeutung haben mit einem Anhydrid der allgemeinen Formel 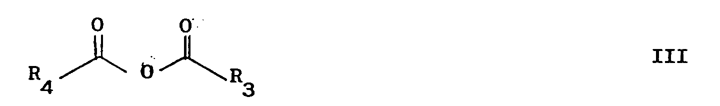 worin R₃ die genannte Bedeutung hat und R₄ eine C₁-C₄-Alkylgruppe bedeutet, in das Produkt gemäss Formel I überführt.

2. Verfahren nach Patentanspruch 1, dadurch gekennzeichnet, dass als Propandiimidat der allgemeinen Formel II Dimethyl-1,3-propandiimidat, worin R₁ und R₂ eine Methylgruppe bedeuten, verwendet.

3. Verfahren nach einem der Patentansprüche 1 und 2, dadurch gekennzeichnet, dass man als Anhydrid der allgemeinen Formel III entweder Formylacetat, worin R₃ ein Wasserstoffatom und R₄ eine Methylgruppe bedeutet, oder Essigsäureanhydrid, worin R₃ und R₄ eine Methylgruppe bedeuten, verwendet.

4. Verfahren nach mindestens einem der Patentansprüche 1 bis 3, dadurch gekennzeichnet, dass man die Umsetzung in Gegenwart einer Base durchführt.

5. Verfahren nach mindestens einem der Patentansprüche 1 bis 4, dadurch gekennzeichnet, dass man die Umsetzung in einem inerten Lösungsmittel durchführt.

6. Verfahren nach mindestens einem der Patentansprüche 1 bis 5, dadurch gekennzeichnet, dass man die Umsetzung bei einer Temperatur von 0 bis 100°C durchführt.
